# FASCICULE DE BREVET EUROPEEN

(11) **EP 0 862 393 B1**
(45) Date de publication et mention de la délivrance du brevet: **12.03.2003**
(21) Numéro de dépôt: 97940205.4
(22) Date de dépôt: 10.09.1997
(51) Int. Cl.: A61F 2/14, A61L 27/00

(54) **BILLE INTRA-ORBITAIRE**
KUGELFÖRMIGE ORBITAPROTHESE
INTRAORBITAL SPHERULE

(30) Priorité: 11.09.1996 FR 9611066
(43) Date de publication de la demande: 09.09.1998
(73) Titulaire: F.C.I. (FRANCE CHIRURGIE INSTRUMENTATION), F-92130 Issy Les Moulineaux (FR)
(72) Inventeur: GUENA, Nicolas, F-92150 Suresnes (FR); AUVERT, Sylvain, F-92100 Boulogne Billancourt (FR); CHAPUT, Christophe, F-87000 Limoges (FR)
(74) Mandataire: Faber, Jean-Paul
(86) Numéro de dépôt international: FR9701590
(87) Numéro de publication internationale: WO98010715

(56) Documents cités:
- WO-A-91/07930
- US-A- 2 688 139
- US-A- 4 000 525
- US-A- 4 976 731
- US-A- 5 549 123

## Description

La présente invention a pour objet une bille destinée à constituer un implant ou prothèse de remplacement d'un globe oculaire après énucléation ou éviscération.

Au cours d'une opération d'énucléation, tout le globe est extrait de la cavité orbitaire. Dans une éviscération, seul le contenu du globe oculaire est extrait, la coque sclérale restant en place.

Dans les deux cas, pour ne pas laisser la cavité orbitaire vide, il est connu depuis longtemps d'insérer une prothèse synthétique de forme sphérique correspondant à la forme de l'oeil normal, soit directement dans la cavité orbitaire dans le cas d'une énucléation, soit dans la coque sclérale dans le cas d'une éviscération.

En avant de cette prothèse synthétique est placé ce qui est communément appelé un "oeil de verre" et qui est en fait une demi-coque en plastique sur laquelle sont dessinés un iris et une pupille, cette coque n'ayant qu'un but esthétique.

Le problème posé par ces anciennes prothèses tient à ce qu'elles étaient constituées d'un matériau biocompatible mais non colonisable comme le plastique (PMMA) ou le silicone ce qui ne permettait pas de relier la prothèse à la demi-coque.

Il est également connu de réaliser un implant intra-orbitaire comprenant un noyau de silicone recouvert d'un tissu biocolonisable tel que du PTFE expansé. L'inconvénient d'une telle prothèse est que la colonisation ne peut se faire qu'en périphérie là où se trouve le tissu biocolonisable interdisant également une liaison entre un tel implant et la coque en plastique.

Dans le brevet US-A-4,976,731 (PERRY), il est proposé de réaliser la bille en céramique poreuse d'hydroxyapatite stérile, obtenue soit à partir de corail, soit par synthèse. La porosité de cette prothèse en hydroxyapatite autorise la colonisation de l'implant dans sa totalité. Cette colonisation permettra de percer ultérieurement un trou à l'intérieur de la bille permettant l'introduction d'un entraîneur reliant la bille avec la demi-coque en plastique. L'entraîneur permet une meilleure transmission des mouvements de la bille à la coque en plastique autorisant une restitution des mouvements de l'oeil artificiel équivalents à ceux de l'oeil adelphe.

Cependant, la fabrication d'un tel implant présente une grande difficulté de mise en oeuvre alliée à un coût de production très élevé.

A cela s'ajoute une solubilité de l'hydroxyapatite dans l'organisme de l'ordre de 3% qui peut être à l'origine de réactions inflammatoires.

On connaît, également, par le brevet US-5,549,123 des implants biocompatibles en général réalisés en céramique frittée à base d'une poudre métallique. Toutefois, ce brevet ne vise pas des implants intra-orbitaire qui doivent répondre à un certain nombre de critères, à savoir notamment présenter une surface la plus lisse possible, avoir un poids acceptable, être biocompatibles et non friables.

La présente invention a pour objet de pallier ces inconvénients, de permettre un abaissement du coût de tels implants et de proposer un matériau à forte innocuité beaucoup plus stable dans le temps.

Selon l'invention, la bille intra-orbitaire utilisable pour remplacer l'oeil en cas d'énucléation ou d'éviscération est du type réalisée à partir d'une céramique frittée obtenue à base de poudre d'alumine et dans laquelle les pores de la céramique sont interconnectés sans obturations et d'un diamètre d'environ 100 microns, la bille intra-orbitaire étant caractérisée en ce que la poudre d'aluminium comporte un ajout de frittage constitué d'oxyde de magnésium dans la proportion de 1% en poids.

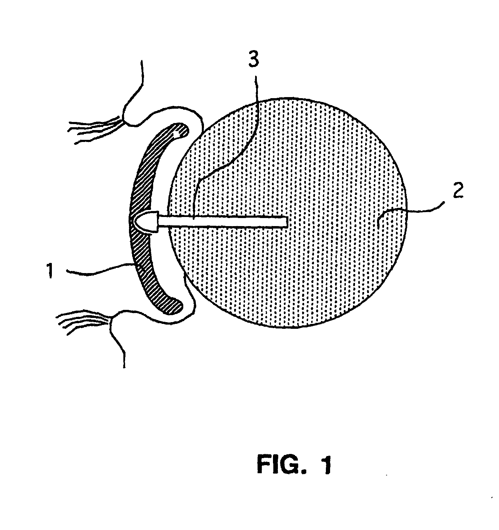

D'autres caractéristiques et avantages de l'invention apparaîtront au cours de la description qui va suivre, donnée uniquement à titre d'exemple non limitatif en regard de la figure unique qui représente schématiquement un implant selon l'invention.

Sur la figure on distingue la bille poreuse colonisable 2 sur laquelle est fixée une demi-coque 1 à l'aide d'un entraîneur 3 constitué par une tige dont une extrémité est fixée à l'intérieur de la bille et dont la seconde extrémité est articulée sur la demi-coque.

La bille est constituée à partir de poudré d'oxyde métallique frittée à pores totalement interconnectés de section sensiblement constante. Une colonisation optimale semble être obtenue avec des pores d'environ 300µ mais elle peut se produire avec des pores plus petits ou plus grands. La bille , une fois colonisée par l'organisme, pourra être percée et recevoir l'entraîneur 3 de la même manière qu'une bille en hydroxyapatite.

La céramique métallique est constituée par de l'alumine en poudre, à laquelle est adjoint un ajout de frittage constitué d'oxyde de magnésium (MgO) dans une proportion de 1% en poids.

L'alumine présente outre une grande disponibilité sur le marché à l'état stoechiométrique ultra pur, une inertie chimique et biologique absolue, et aucune solubilité n'est notée. Par ailleurs, l'alumine frittée présente une très grande résistance mécanique.

La préparation de l'alumine frittée est effectuée à partir d'une poudre d'alumine de granulométrie déterminée et d'une mousse synthétique. La mousse est imprégnée par une barbotine et on lui fait subir un frittage. Le chauffage fait fondre le plastique et agglomère l'alumine en laissant subsister des canaux ou pores de la mousse. Les diamètres des pores sont fonction des pores de la mousse d'origine.

L'inconvénient que pourrait présenter la céramique d'alumine frittée réside dans sa très grande résistance mécanique ce qui la rend difficilement perçable. Ce problème cependant est résolu avec l'alumine poreuse en utilisant un ajout de frittage, constitué d'oxyde de magnésium (MgO) dans une proportion de 1% en poids, ce qui permet d'abaisser la température de frittage de l'ordre de 100°C (1500°C au lieu de 1600°C) et, ainsi, de réduire les propriétés mécaniques de l'alumine la rendant plus facilement perçable à l'aide d'une perceuse et d'une fraise six pans.

## Revendications

1. Bille intra-orbitaire constituant un implant après énucléation ou éviscération, constituée par une céramique frittée poreuse à base de poudre d'alumine et dans laquelle les pores de la céramique sont interconnectés et sans obturations d'un diamètre de 300 microns environ, **caractérisée en ce que** la poudre d'alumine comporte un ajout de frittage constitué d'oxyde de magnésium (MgO) dans une proportion de 1% en poids.

## Claims

1. An intra-orbital sphere forming an implant after enucleation or evisceration, formed by a porous sintered ceramic material on a base of aluminium oxide powder and in which the pores of the ceramic material are interconnected and have no dead ends and a diameter of roughly 300 microns,
**characterised in that** the aluminium oxide powder has a sintering addition of formed of magnesium oxide (MgO) in a proportion of 1 % by weight.

## Patentansprüche

1. Kugel innerhalb der Augenhöhle, ein Implantat nach Ausschälung oder Ausweidung bildend, bestehend aus einer gesinterten, porösen Keramik auf Grundlage von Aluminiumoxidpulver, bei der die Poren der Keramik untereinander verbunden und ohne Verstopfungen sind, bei einem Durchmesser von etwa 300 Mikrometern, **dadurch gekennzeichnet, daß** das Aluminiumoxidpulver einen Sinterzusatz, bestehend aus Magnesiumoxid (MgO) in einem Anteil von 1 Gewichts.-Prozent enthält.
